Europäisches Patentamt

European Patent Office

Office européen des brevets

(11) Publication number: **0 227 108 B1**

## EUROPEAN PATENT SPECIFICATION

(45) Date of publication of patent specification: **31.03.93**  (51) Int. Cl.5: **A61K 7/26**, A61K 7/22

(21) Application number: **86117985.1**

(22) Date of filing: **23.12.86**

(54) **Oral composition.**

(30) Priority: **27.12.85 JP 299157/85**
**03.09.86 JP 207590/86**

(43) Date of publication of application:
**01.07.87 Bulletin 87/27**

(45) Publication of the grant of the patent:
**31.03.93 Bulletin 93/13**

(84) Designated Contracting States:
**DE ES FR GB**

(56) References cited:
**JP-A- 5 756 415**

**PATENT ABSTRACTS OF JAPAN, vol. 6, no.
169 (C-122)[1047], 29 October 1982**

**PATENT ABSTACTS OF JAPAN, vol. 10, no.
194 (C-358)[2250], 08 July 1986**

**PATENT ABSTRACTS OF JAPAN, vol. 7, no.
219 (C-188)[1364], 29 September 1983**

(73) Proprietor: **LION CORPORATION**
**3-7, Honjo 1-chome**
**Sumida-ku Tokyo(JP)**

(72) Inventor: **Fukuchi, Naoji**
**1-14-14, Osu**
**Ichikawa-shi Chiba(JP)**
Inventor: **Suganuma, Nobuo**
**1-3-25, Yashiki**
**Narashino-shi Chiba(JP)**
Inventor: **Yoshida, Fumio**
**4-25-8, Shimoigusa**
**Suginami-ku Tokyo(JP)**
Inventor: **Matsumoto, Tamami**
**25-4, Mitsusawa-kamicho**
**Yokohama-shi Kanagawa(JP)**

(74) Representative: **Henkel, Feiler, Hänzel & Partner**
**Möhlstrasse 37**
**W-8000 München 80 (DE)**

EP 0 227 108 B1

**Description**

BACKGROUND OF THE INVENTION

1. Field of the invention

The present invention relates to an oral composition containing berberine and a flavour composition containing aldehydes. More specifically, the present invention relates to an oral composition capable of stably retaining berberine over a long time.

2. Description of the Related Art

The formulation of a galenical extract such as coptis extract, phellodendron extract, etc., containing berberine in an oral composition is well known in the prior art (for example, Japanese Unexamined Patent Publication (Kokai) Nos. 57-56415, 57-85319, 58-39615, and 58-57320). These berberine-containing galenical extracts have antiinflammatory and antibacterial actions, and further, exhibit the effects of obstructing the attachment of microorganism cells to teeth surfaces, inhibiting the formation of plaque, and preventing mouth odor, when formulated in an oral composition. Nevertheless, the galenical extract containing berberine has a bitter taste caused by the berberine base, and a problem arises in that discomfort is felt during use of the oral composition, particularly the taste is unpleasant.

Furthermore, to obtain the antiinflammatory effect, the antibacterial effect, and the effect of obstructing attachment of microorganisms within the mouth to teeth surfaces, it has been proposed that berberine or a galenical extract containing berberine such as coptis extract, phellodendron extract, or scutellaria extract, be formulated in an oral composition (see Japanese Unexamined Patent Publication (Kokai) Nos. 57-56415, 57-85319, 58-39615, and 58-57320).

However, when berberine is formulated in an oral composition such as dentifrice, and the composition is stored for a long term, a problem arises in that the residual percentage of berberine will be lowered. For this reason, a stabilized formulation of berberine into an oral composition is desired.

JP-A-61-36215 discloses monoterpene alcohols and ketones as flavour components in oral compositions. However, neither the use of special aldehyde flavours in such oral compositions is described nor their stabilizing effects for berberine compositions.

SUMMARY OF THE INVENTION

Accordingly, the objects of the present invention are to eliminate the above-mentioned disadvantages of the prior art and to provide an oral composition capable of stably retaining berberine over a long term.

Other objects and advantages of the present invention will be apparent from the following description.

In accordance with the present invention, there is provided an oral composition comprising berberine and a flavour composition containing at least one aldehyde flavour selected from the group consisting of butanal, iso-butylaldehyde, pivalaldehyde, 2-methyl-pentanal, 2,3,5,5-tetramethylhexanal, 2-methyldecanal, tetradecanal, anisaldehyde, iso-valeric aldehyde, p-veratric aldehyde, and cinnamic aldehyde, whereas the content of straight chain aldehyde compounds having 5 to 12 carbon atoms in the flavour composition is 0.001% by weight or less, based on the total weight of the oral composition.

Also in accordance with the present invention, there is provided an oral composition comprising berberine and a flavour composition as described above which comprises a nonionic surfactant. A further aspect of the invention is a process for the preparation of the above mentioned compositions, wherein a flavour composition containing at least one aldehyde flavour selected from the group consisting of butanal, iso-butylaldehyde, pivalaldehyde, 2-methyl-pentanal, 2,3,5,5-tetramethylhexanal, 2-methyldecanal, tetradecanal, anisaldehyde, iso-valeric aldehyde, p-veratric aldehyde, and cinnamic aldehyde is added to the berberine composition, whereby the content of a flavour composition containing straight chain aldehyde compounds having 5 to 12 carbon atoms is controlled to not more than 0.001% by weight based on the total weight of the oral composition.

DESCRIPTION OF THE PREFERRED EMBODIMENTS

The oral composition containing berberine according to the present invention may be prepared and applied as dentifrices such as toothpaste, wet dentifrice, and liquid dentifrice, mouth wash, troach, chewing gum, oral paste, gingiva massage cream, liquid mouth refrigerant, and solid mouth refrigerant.

Berberine can be formulated in the present invention as a galenical extract containing berberine, such as phellodendron extract, coptis extract, and scutellaria extract. Among these, phellodendron extract is preferred. It is also preferred that the amount of the berberine-containing galenical extract be 0.001% to 3% by weight (hereinafter all prcentages are by weight unless otherwise stated) of the total oral composition. In this amount, berberine is contained at a concentration of about 1 to 3000 ppm.

According to the present invention, the stability of the berberine in the oral composition can be obtained by controlling the content of the straight chain aldehyde compounds having 5 to 12 carbon atoms contained in the flavour composition to 0.001% or less of the otal oral composition.

Thus, the present inventors have carried out intensive studies into a stabilized formulation of berberine into an oral composition, and consequently, found that the stability of the berberine is impaired by the aldehyde type flavours contained in the flavour composition to be formulated for flavouring the oral composition. Nevertheless, as a result of further progress in our detailed investigations, it has been found that some of the aldehyde type flavours impair the stability of the berberine, but some retain the stability of berberine substantially without impairment. The flavours which impair berberine are straight chain aldehyde compounds having 5 to 12 carbon atoms such as pentanal, n-hexylaldehyde, heptanal, n-octylaldehyde, n-nonylaldehyde, undecanal, and dodecanal. By ensuring that these aldehyde compounds are not contained substantially in the oral composition formulated with berberine, more specifically, by controlling their amounts to a level of not more than 0.001% in the oral composition, a stabilized formulation of berberine is accomplished, whereby the effect of berberine can be effectively exhibited even after storage for a long time. Further, the aldehyde type flavours which do not impair the stability of berberine have been found to be butanal, iso-butylaldehyde, pivalaldehyde, 2-methylpentanal, 2,3,5,5-tetramethylhaxanal, 2-methyldecanal, tetradecanal, anisaldehyde, iso-valeric aldehyde, p-veratric aldehyde, and cinnamic aldehyde. When one or more of these aldehydes are formulated, it has been found that the specific taste, i.e., bitterness or smell of berberine can be masked to give an oral composition which does not cause discomfort during usage.

In the berberine-formulated oral composition of the present invention, a flavour composition is formulated for adding flavour to improve the feeling during usage. In the present invention, when the flavour composition is formulated into the oral composition, a flavour composition is employed which is controlled so that straight chain aldehyde compounds having 5 to 12 carbon atoms are not substantially contained in the berberine-formulated oral composition, specifically to an existing amount of not more than 0.001%, preferably not more than 0.0001%. For example, for flavouring an oral composition, a natural essential oil such as peppermint oil or spearmint oil is frequently used, and about 0.01 to 3% of aldehyde compounds having 5 to 12 carbon atoms are ordinarily contained in these essential oils. Accordingly, when these essential oils are employed, they are used in an amount such that the content of the straight chain aldehyde compounds having 5 to 12 carbon atoms in the oral composition may be not more than 0.001%. If the content of the straight chain aldehyde compounds having 5 to 12 carbon atoms in the oral composition is more than 0.001%, a stabilized formulation of the berberine is unpreferably impaired.

The flavour composition usable in the present invention is not particularly limited, except that the amount of the straight chain aldehyde compounds having 5 to 12 carbon atoms is regulated as described above, and it can be made up with the use of one or more of various flavour components including, for example, essential oils such as peppermint oil, spearmint oil, wintergreen oil, rose oil, cardamon oil, clove oil, orange oil, thyme oil, etc., and single flavours such as ℓ-menthol, cineole, anethole, ℓ-carvone, and eugenol. Also, aldehyde type flavours other than the straight chain aldehyde compounds having 5 to 12 carbon atoms can be formulated. However, as the aldehyde type flavour, and from the viewpoint of reducing the impairment of the stability of the berberine, butanal, iso-butylaldehyde, pivalaldehyde, 2-methylpentanal, 2,3,5,5-tetramethylhexanal, 2-methyldecanal, tetradecanal, anisaldehyde, iso-valeric aldehyde, p-veratric aldehyde and cinnamic aldehyde are specifically claimed and one kind or two or more kinds of these may be used. By formulation of these aldehyde flavours, the specific taste (bitterness) or smell of berberine can be masked to improve the feeling during usage of the berberine-formulated oral composition. In this case, the amount of these aldehyde flavours formulated may be preferably not less than 0.1% of the flavour composition. The amount of the flavour composition formulated in the oral composition is not particularly limited, but is generally 0.1 to 10%.

According to the second aspect of the present invention, the berberine-formulated oral composition can stably retain berberine over a long term to effectively inhibit the effect of berberine by controlling the content of the straight chain aldehyde compounds having 5 to 12 carbon atoms contained in the flavour composition to not more than 0.001% of the total oral composition.

The oral composition according to the present invention can further contain optional components, in addition to the components as described above, depending upon, for example, the kind and the purpose of use, such as abrasives, binders, tackifiers, flavours, sweeteners, preservatives, and various effective ingredients, and it can be prepared according to a conventional manner.

According to a further aspect of the present invention, a nonionic surfactant is formulated in the above oral composition to inhibit the bitterness derived from berberine. Examples of such nonionic surfactants are alkanolamide fatty acid esters, sucrose fatty acid esters, lactitol fatty acid esters, multitol fatty acid esters, glycerine fatty acid esters, sorbitane fatty acid esters, polyoxyethyleneglycerine fatty acid esters, polyoxyethylenesorbitane fatty acid esters, polyoxyethylene hydrogenated castor oil, and polyoxyethylene fatty acid esters. These surfactants may be used alone or in any mixture thereof. To obtain a more effective inhibition of the bitterness of berberine, a fatty acid alkanolamide is most preferably used.

The alkanolamide fatty acid esters usable in the present invention are those with fatty acid groups having 9 to 18 carbon atoms, and alkanol groups having 2 or 3 carbon atoms are preferred. The fatty acid may be either saturated or unsaturated, and may be either straight or have a branched chain, or further, may be a mixed fatty acid. More specifically, there may be employed caproyl monoethanolamide, lauroyl diethanolamide, myristoyl diethanolamide, palmitoyl diethanolamide, coconut fatty diethanolamide, tallow fatty diethanolamide, and lauroyl monoisopropanolamide, etc. Among these, lauroyl diethanolamide and myristoyl diethanolamide are preferably used.

The amount of the nonionic surfactant formulated may be 0.1 to 5%, particularly 0.2 to 3%. When formulated at a level of less than 0.1%, the effect of lowering the bitterness of berberine may not always be sufficient, and with an amount exceeding 5%, in some causes the foaming speed may be retarded to worsen the discomfort felt during usage.

According to the present invention, in addition to the above-mentioned nonionic surfactant, it is also possible to formulate one or more other surfactants such as anionic surfactants, including water-soluble salts of alkylsulfates having 8 to 18 carbon atoms such as sodium lauryl sulfate, and sodium myristoyl sulfate; water-soluble sulfuric acid salts of higher fatty acid monoglycerides with fatty acid groups having 10 to 18 carbon atoms such as sodium lauryl monoglyceride sulfate, sodium coconut fatty monoglyceride sulfate, and sodium higher fatty acid monoglyceride monosulfate, sodium salts of $\alpha$-olefin sulfonate, paraffin sulfonate, N-methyl-N-palmitoyl tauride, sodium lauroyl sarcosinate, and sodium N-lauroyl-$\beta$-alanine, as well as cationic surfactants and amphoteric surfactants.

Any bitterness in the oral composition can be inhibited, to remarkably improve the discomfort felt during usage, by formulating a nonionic surfactant, particularly an alkanolamide fatty acid ester in the oral composition containing berberine.

Examples

The present invention will now be further illustrated by the following Examples, wherein all parts and percentages are expressed on a weight basis unless otherwise noted.

Evaluation Example 1 and Examples 1 to 15 are present for illustrative purposes only; the subject-matter of which is not claimed per se.

Evaluation Example 1

A toothpaste having the following recipe was prepared, and the effect thereof of reducing bitterness was evaluated organoleptically by a panel of experts.

Here, the discomfort felt during usage was judged by the taste when about 2 g of the toothpaste was put on a tooth brush and used in a conventional manner. The results are shown in Table 1.

4

| Toothpaste recipe | |
|---|---|
| Dicalcium phosphate | 45.0% |
| Precipitated silica | 2.0 |
| Propylene glycol | 2.5 |
| Sorbitol | 25.0 |
| Sodium carboxymethyl cellulose | 0.5 |
| Carrageenan | 0.5 |
| Sodium lauryl sulfate | 0.5 |
| Lauroyl diethanolamide | Amount indicated in Table 1 |
| Phellodendron aqueous extract (containing about 5% berberine) | 0.1 |
| Flavour | 0.5 |
| Sodium saccharin | 0.1 |
| Purified water | balance |
| | 100.0% |

Table 1

| | 1 | 2 | 3 | 4 | 5 |
|---|---|---|---|---|---|
| Amount of lauroyl diethanolamide formulated | 0% | 0.05 | 0.1 | 0.5 | 1.0 |
| Result of judgment | x | △ | o | o | o |
| The evaluation standards are as shown below. o: no bitterness. △: slight bitterness. x: bitterness. | | | | | |

From the results shown in Table 1, it was recognized that the bitterness caused by the berberine base could be avoided by the formulation of lauroyl diethanolamide (nonionic surfactant).

Evaluation Example 2

When dicalcium phosphate is used as the abrasive, 0.5% of an aldehyde type single flavour as indicated in Table 2 was formulated into the toothpaste of the recipe shown below formulated with phellodendron extract (containing about 5% berberine), and the dentifrice sample was filled into a tube and stored in a thermostat layer at 60°C for 5 days for examination of the storage stability (residual percentage) of berberine. The results are shown in Table 2. The method for measuring berberine is as described below.

| Toothpaste recipe | |
|---|---|
| Dicalcium phosphate | 45.0% |
| Precipitated silica | 2.0 |
| Propylene glycol | 2.5 |
| Sorbitol | 25.0 |
| Sodium carboxymethyl cellulose | 0.5 |
| Carrageenan | 0.5 |
| Sodium lauryl sulfate | 0.5 |
| Lauroyl diethanolamide | 0.3 |
| Phellodendron extract (containing about 5% berberine) | 0.1 |
| Aldehyde type single flavour | 0.5 |
| Sodium saccharin | 0.1 |
| Purified water | balance |
| | 100.0% |

Berberine determination method

An amount of 5.0 g of toothpaste sample was dispersed in 50 ml of an extract (i.e., tartaric acid solution + $CH_3OH$ + $CH_3CN$), the supernatent was collected after centrifugation at 10000 rpm for 10 minutes, and the residual percentage of berberine was measured by liquid chromatography.

Table 2

| Aldehyde type single flavour | Berberine residual percentage (%) |
|---|---|
| Butanal | 99.1 |
| iso-Butylaldehyde | 88.1 |
| Pentanal | 19.4 |
| 2-Methylbutanal | 69.3 |
| Pivalaldehyde | 95.6 |
| n-Hexylaldehyde | 0 |
| 2-Methylpentanal | 76.8 |
| Heptanal | 0 |
| n-Octylaldehyde | 0 |
| n-Nonylaldehyde | 0 |
| Decanal | 52.1 |
| 2,3,5,5-Tetramethylhexanal | 81.0 |
| Undecanal | 17.3 |
| 2-Methyldecanal | 75.9 |
| Dodecanal | 22.3 |
| 2-Methylundecanal | 63.9 |
| Tetradecanal | 101.2 |
| Anisaldehyde | 90.3 |
| iso-Valeric aldehyde | 96.8 |
| p-Veratric aldehyde | 100.4 |
| Cinnamic aldehyde | 90.7 |

From the results in Table 2, it has been found that straight chain aldehyde compounds having 5 to 12 carbon atoms will remarkably lower the residual percentage of berberine, and also that butanal, iso-butylaldehyde, pivalaldehyde, 2-methylpentanal, 2,3,5,5-tetramethylhexanal, 2-methyldecanal, tetradecanal, anisaldehyde, iso-valeric aldehyde, p-veratric aldehyde, and cinnamic aldehyde stably retain berberine.

The formulation Examples are shown below.

| Example 1: Toothpaste | |
| --- | --- |
| Dicalcium phosphate | 45.0% |
| Precipitated silica | 2.0 |
| Sodium carboxymethyl cellulose | 0.5 |
| Carrageenan | 0.5 |
| Sorbitol | 25.0 |
| Glycerol | 15.0 |
| Propylene glycol | 3.0 |
| Sodium lauryl sulfate | 0.5 |
| Lauroyl diethanolamide | 0.5 |
| Sodium lauroyl sarcosinate | 0.5 |
| $\alpha$-Tocopherol acetate | 0.1 |
| Tranexamic acid | 0.05 |
| $\beta$-Glycyrrhetinic acid | 0.01 |
| Phellodendron aqueous extract | 0.1 |
| Methyl parahydroxybenzoate | 0.1 |
| Sodium benzoate | 0.5 |
| Sodium saccharin | 0.1 |
| Flavour | 1.0 |
| Purified water | balance |
| | 100.0% |

## Example 2:  Toothpaste

| | |
| --- | --- |
| Dicalcium phosphate | 50.0% |
| Sodium carboxymethyl cellulose | 1.0 |
| Sorbitol | 15.0 |
| Glycerol | 15.0 |
| Propylene glycol | 2.0 |

| | |
|---|---|
| Ethyl alcohol | 0.5 |
| Sodium lauryl sulfate* | 0.5 |
| Sodium lauroyl sarcosinate | 0.3 |
| Lauroyl diethanolamide | 1.0 |
| α-Tocopherol acetate | 0.05 |
| ε-Aminocaproic acid | 0.03 |
| β-Glycyrrhetinic acid | 0.01 |
| Phellodendron extract | 0.1 |
| Isopropyl methyl phenol | 0.01 |
| Ethyl parahydroxybenzoate | 0.1 |
| Sodium benzoate | 0.5 |
| Sodium saccharin | 0.1 |
| Flavour | 1.0 |
| Purified water | balance |
| | 100.0% |

Sodium lauryl sulfate*

$C_{10}$:    0 - 12%

$C_{12}$:    50 - 80

$C_{14}$:    10 - 30

$C_{16}$:    0 - 15

The above components account for 98% or more of the total carbons. This is also the case in the following formulations.

8

| Example 3: Toothpaste | |
|---|---|
| Calcium carbonate | 45.0% |
| Sodium carboxymethyl cellulose | 1.5 |
| Sorbitol | 25.0 |
| Glycerol | 10.0 |
| Propylene glycol | 3.0 |
| Sodium lauryl sulfate | 2.0 |
| Lauroyl diethanolamide | 1.0 |
| Myristoyl diethanolamide | 0.5 |
| Dipotassium glycyrrhizinate | 0.01 |
| Sodium chloride | 7.0 |
| Coptis aqueous extract | 0.1 |
| Ethyl parahydroxybenzoate | 0.03 |
| Sodium benzoate | 1.0 |
| Sodium saccharin | 0.1 |
| Flavour | 1.0 |
| Purified water | balance |
| | 100.0% |

| Example 4: Toothpaste | |
|---|---|
| Aluminum hydroxide | 40.0% |
| Carrageenan | 1.0 |
| Sorbitol | 30.0 |
| Propylene glycol | 3.0 |
| Sodium lauryl sulfate | 1.5 |
| Lauroyl diethanolamide | 1.5 |
| Palmitoyl diethanolamide | 0.5 |
| Tranexamic acid | 0.05 |
| Chlorhexidine hydrochloride | 0.001 |
| Scutellaria aqueous extract | 0.1 |
| Ethyl parahydroxybenzoate | 0.05 |
| Sodium benzoate | 0.3 |
| Sodium saccharin | 0.1 |
| Flavour | 1.0 |
| Purified water | balance |
| | 100.0% |

| Example 5: Toothpaste | |
|---|---|
| Aluminum hydroxide | 45.0% |
| Precipitated silica | 2.0 |
| Carrageenan | 1.0 |
| Sodium alginate | 0.5 |
| Sorbitol | 35.0 |
| Sodium lauryl sulfate* | 1.0 |
| Myristoyl diethanolamide | 1.5 |
| Coconut fatty diethanolamide | 0.3 |
| Dextranase | 2000 U/g |
| Monofluorophosphate | 0.76 |
| Phellodendron extract | 0.05 |
| Methyl parahydroxybenzoate | 0.2 |
| Sodium benzoate | 0.3 |
| Stevioside | 0.1 |
| Flavour | 1.0 |
| Purified water | balance |
| | 100.0% |

| Example 6: Toothpaste | |
|---|---|
| Dicalcium phosphate | 40.0% |
| Xanthan gum | 1.0 |
| Glycerol | 25.0 |
| Propylene glycol | 5.0 |
| Ethyl alcohol | 0.5 |
| Sodium lauryl sulfate* | 1.0 |
| Sodium lauroyl sarcosinate | 0.5 |
| Sucrose monolaurate | 0.1 |
| Sucrose monopalmitate | 0.1 |
| Polyoxyethylene sorbitane monolaurate | 0.1 |
| $\alpha$-Tocopherol acetate | 0.1 |
| Aluminum chlorohydroxy allantoinate | 0.01 |
| Coptis aqueous extract | 0.1 |
| Ethyl parahydroxybenzoate | 0.02 |
| Sodium benzoate | 1.0 |
| Aspartame | 0.1 |
| Flavour | 1.0 |
| Purified water | balance |
| | 100.0% |

| Example 7: Toothpaste | |
|---|---|
| Precipitated silica | 30.0% |
| Carrageenan | 1.5 |
| Sorbitol | 15.0 |
| Glycerol | 25.0 |
| Polyethylene glycol | 5.0 |
| Sodium lauryl sulfate | 1.5 |
| Sodium lauroyl sarcosinate | 0.3 |
| Lauroyl diethanolamide | 0.7 |
| Myristoyl diethanolamide | 0.3 |
| Polyoxyethylene hardened castor oil | 0.3 |
| Tocopherol nicotinate | 0.3 |
| $\beta$-Glycyrrhetinic acid | 0.07 |
| Sodium fluoride | 0.2 |
| Phellodendron extract | 0.005 |
| Methyl parahydroxybenzoate | 0.1 |
| Sodium benzoate | 0.5 |
| Sodium saccharin | 0.1 |
| Flavour | 1.0 |
| Purified water | balance |
| | 100.0% |

| Example 8: Toothpaste | |
|---|---|
| Aluminum hydroxide | 45.0% |
| Carrageenan | 0.3 |
| Hydroxyethyl cellulose | 1.5 |
| Sorbitol | 40.0 |
| Sodium lauryl sulfate* | 0.7 |
| Polyoxyethylene hardened castor oil | 1.0 |
| $\alpha$-Tocopherol acetate | 0.5 |
| $\beta$-Glycyrrhetinic acid | 0.04 |
| Stanous fluoride | 0.4 |
| Phellodendron aqueous extract | 1.0 |
| Butyl parahydroxybenzoate | 0.02 |
| Sodium benzoate | 0.3 |
| Sodium saccharin | 0.1 |
| Flavour | 1.0 |
| Purified water | balance |
| | 100.0% |

| Example 9: Toothpaste | |
|---|---|
| Calcium pyrophosphate | 50.0% |
| Sodium carboxymethyl cellulose | 1.5 |
| Glycerol | 30.0 |
| Sodium lauryl sulfate | 0.5 |
| Lauroyl diethanolamide | 2.0 |
| Myristoyl diethanolamide | 0.5 |
| Tocopherol nicotinate | 0.05 |
| Sodium monofluorophosphate | 0.76 |
| Phellodendron extract | 0.5 |
| Methyl parahydroxybenzoate | 0.05 |
| Sodium benzoate | 0.5 |
| Sodium saccharin | 0.1 |
| Flavour | 1.0 |
| Purified water | balance |
| | 100.0% |

| Example 10: Toothpaste | |
|---|---|
| Insoluble calcium metaphosphate | 50.0% |
| Sodium carboxymethyl cellulose | 1.0 |
| Sorbitol | 10.0 |
| Glycerol | 20.0 |
| Sodium lauryl sulfate | 0.7 |
| Sodium lauroyl sarcosinate | 0.3 |
| Lauroyl diethanolamide | 1.5 |
| $\beta$-Glycyrrhetinic acid | 0.1 |
| Phellodendron extract | 0.05 |
| Scutellaria 30% ethanol extract | 0.05 |
| Butyl parahydroxybenzoate | 0.1 |
| Sodium benzoate | 0.1 |
| Sodium saccharin | 0.1 |
| Flavour | 1.0 |
| Purified water | balance |
| | 100.0% |

| Example 11: Toothpaste | |
| --- | --- |
| Calcium pyrophosphate | 20.0% |
| Sodium carboxymethyl cellulose | 1.0 |
| Carrageenan | 1.0 |
| Glycerol | 40.0 |
| Polyethylene glycol | 3.0 |
| Sodium lauryl sulfate | 2.0 |
| Lauroyl diethanolamide | 1.0 |
| Myristoyl diethanolamide | 0.5 |
| Coconut fatty diethanolamide | 0.5 |
| Palmitoyl diethanolamide | 0.5 |
| $\alpha$-Tocopherol acetate | 0.3 |
| $\epsilon$-Aminocaproic acid | 0.03 |
| $\beta$-Glycyrrhetinic acid | 0.02 |
| Phellodendron aqueous extract | 0.05 |
| Coptis aqueous extract | 0.05 |
| Methyl parahydroxybenzoate | 0.05 |
| Sodium benzoate | 0.5 |
| Sodium saccharin | 0.1 |
| Flavour | 1.0 |
| Purified water | balance |
| | 100.0% |

| Example 12: Wet dentifrice | |
| --- | --- |
| Calcium carbonate | 70.0% |
| Sorbitol | 2.0 |
| Glycerol | 8.0 |
| Sodium lauryl sulfate | 1.5 |
| Lauroyl diethanolamide | 1.0 |
| $\alpha$-Tocopherol acetate | 0.1 |
| Phellodendron extract | 0.05 |
| Sodium saccharin | 0.1 |
| Flavour | 1.0 |
| Purified water | balance |
| | 100.0% |

| Example 13: Liquid dentifrice | |
| --- | --- |
| Glycerol | 35.0% |
| Propylene glycol | 5.0 |
| Sodium polyacrylate | 2.5 |
| Sodium lauryl sulfate | 1.0 |
| Lauroyl diethanolamide | 1.5 |
| Phellodendron extract | 0.5 |
| Sodium saccharin | 0.3 |
| Flavour | 5.0 |
| Purified water | balance |
| | 100.0% |

| Example 14: Mouthwash | |
|---|---|
| Ethyl alcohol (96%) | 20.0% |
| Polyoxyethylene hardened caster oil | 0.5 |
| Sodium lauryl sulfate | 0.5 |
| Lauroyl diethanolamide | 1.0 |
| Phellodendron extract | 0.2 |
| Sodium saccharin | 0.4 |
| Flavour | 3.0 |
| Purified water | balance |
| | 100.0% |

| Example 15: Pasta | |
|---|---|
| Hydroxyethyl cellulose | 4.0% |
| Sorbitol | 40.0 |
| Sodium lauryl sulfate | 0.5 |
| Lauroyl diethanolamide | 2.0 |
| Phellodendron extract | 0.4 |
| Methyl parahydroxybenzoate | 0.02 |
| Sodium saccharin | 0.02 |
| Flavour | 1.0 |
| Purified water | balance |
| | 100.0% |

Bitterness in all of the oral compositions of the Examples 1 to 15 was inhibited and no discomfort was caused during usage.

The compositions of the flavours used in the following Examples are shown in Table 3.

Table 3

| Starting material: | Flavour No. | | | | |
|---|---|---|---|---|---|
| | I | II | III | IV | V |
| $\ell$-Menthol | 40.0 | 40.0 | 25.0 | 20.0 | 20.0 |
| Cineole | - | 30.0 | 3.0 | 2.0 | 5.0 |
| Anethole | 15.0 | 10.0 | 30.0 | 8.0 | 15.0 |
| Peppermint oil | 20.0 | 5.0 | 10.0 | 15.0 | - |
| Spearmint oil | - | - | - | 10.0 | - |
| $\ell$-Carvone | 2.0 | - | 1.0 | 30.0 | 7.0 |
| Wintergreen oil | 1.0 | - | 1.0 | 1.0 | 30.0 |
| Rose oil | - | - | 0.2 | - | 0.2 |
| Cardamon oil | 0.2 | 0.5 | 0.2 | - | - |
| Clove oil | 0.1 | 0.5 | - | 0.5 | 0.5 |
| Eugenol | - | 0.5 | 0.5 | 0.5 | 0.5 |
| $\ell$-Menthyl acetate | 10.0 | 2.0 | 5.0 | - | 2.0 |
| Oragne oil | - | 0.5 | 0.5 | 0.5 | - |
| Thyme oil | - | - | - | 0.5 | 0.2 |
| Anisaldehyde | 1.0 | - | 2.0 | - | 1.0 |
| Cinnamic aldehyde | - | 2.0 | 0.2 | 0.5 | 3.0 |
| Isovareloaldehyde | - | - | - | 0.05 | - |
| Ethanol | balance | balance | balance | balance | balance |
| Total | 100 parts | 100 parts | 100 parts | 100 parts | 100 parts |
| Content of $C_5$-$C_{12}$ straight chain aldehyde compounds | < 100 ppm | < 100 ppm | < 100 ppm | < 100 ppm | < 100 ppm |

| Example 16: Toothpaste | |
|---|---|
| Dicalcium phosphate | 45.0% |
| Precipitated silica | 2.0 |
| Sodium carboxymethyl cellulose | 0.5 |
| Carrageenan | 0.5 |
| Sorbitol | 25.0 |
| Glycerol | 15.0 |
| Propylene glycol | 3.0 |
| Sodium lauryl sulfate | 0.5 |
| Sodium lauroyl sarcosinate | 0.5 |
| Lauroyl diethanolamide | 0.5 |
| α-Tocopherol acetate | 0.1 |
| Tranexamic acid | 0.05 |
| β-Glycyrrhetinic acid | 0.01 |
| Phellodendron aqueous extract | 0.1 |
| Methyl parahydroxybenzoate | 0.1 |
| Sodium benzoate | 0.5 |
| Sodium saccharin | 0.1 |
| Flavour composition No. I | 0.5 |
| Purified water | balance |
| | 100.0% |

Example 17: Toothpaste

Dicalcium phosphate                50.0%

Sodium carboxymethyl cellulose      1.0

Sorbitol                           15.0

Glycerol                           15.0

Propylene glycol                    2.0

Ethyl alcohol                       0.5

Sodium alkyl sulfate*               0.5

Sodium lauroyl sarcosinate          0.3

Lauroyl diethanolamide              1.0

α-Tocopherol acetate                0.05

ε-Aminocaproic acid                 0.03

β-Glycyrrhetinic acid               0.01

16

| | |
|---|---|
| Phellodendron extract | 0.1 |
| Isopropyl methyl phenol | 0.01 |
| Ethyl parahydroxybenzoate | 0.1 |
| Sodium benzoate | 0.5 |
| Sodium saccharin | 0.1 |
| Flavour composition No. V | 1.0 |
| Purified water | balance |
| | 100.0% |

\* Sodium alkyl sulfate

The following components account for 98% or more of the total carbons. (This is also the case in the following formulations.)

| | |
|---|---|
| $C_{10}$ | 0 - 12% |
| $C_{12}$ | 50 - 80 |
| $C_{14}$ | 10 - 30 |
| $C_{16}$ | 0 - 15 |

| Example 18: Toothpaste | |
|---|---|
| Calcium carbonate | 45.0% |
| Sodium carboxymethyl cellulose | 1.5 |
| Sorbitol | 25.0 |
| Glycerol | 10.0 |
| Propylene glycol | 3.0 |
| Sodium lauryl sulfate | 2.0 |
| Lauroyl diethanolamide | 1.0 |
| Myristoyl diethanolamide | 0.5 |
| Dipotassium glycyrrhizinate | 0.01 |
| Sodium chloride | 7.0 |
| Coptis extract | 0.1 |
| Ethyl parahydroxybenzoate | 0.03 |
| Sodium benzoate | 1.0 |
| Sodium saccharin | 0.1 |
| Flavour composition No. V | 1.0 |
| Purified water | balance |
| | 100.0% |

17

| Example 19: Toothpaste | |
|---|---|
| Aluminum hydroxide | 40.0% |
| Carrageenan | 1.0 |
| Sorbitol | 30.0 |
| Propylene glycol | 3.0 |
| Sodium lauryl sulfate | 1.5 |
| Lauroyl diethanolamide | 1.5 |
| Palmitoyl diethanolamide | 0.5 |
| Tranexamic acid | 0.05 |
| Chlorhexidine hydrochloride | 0.001 |
| Scutellaria aqueous extract | 0.1 |
| Ethyl parahydroxybenzoate | 0.05 |
| Sodium benzoate | 0.3 |
| Sodium saccharin | 0.1 |
| Flavour composition No. IV | 1.0 |
| Purified water | balance |
| | 100.0% |

| Example 20: Toothpaste | |
|---|---|
| Aluminum hydroxide | 45.0% |
| Precipitated silica | 2.0 |
| Carrageenan | 1.0 |
| Sodium alginate | 0.5 |
| Sorbitol | 35.0 |
| Sodium alkyl sulfate* | 1.0 |
| Myristoyl diethanolamide | 1.5 |
| Coconut fatty diethanolamide | 0.3 |
| Dextranase | 2000 u/g |
| Sodium monofluorophosphate | 0.76 |
| Phellodendron extract | 0.05 |
| Methyl parahydroxybenzoate | 0.2 |
| Sodium benzoate | 0.3 |
| Stevioside | 0.1 |
| Flavour composition No. III | 1.0 |
| Purified water | balance |
| | 100.0% |

18

| Example 21: Toothpaste | |
|---|---|
| Dicalcium phosphate | 40.0% |
| Xanthan gum | 1.0 |
| Glycerol | 25.0 |
| Propylene glycol | 5.0 |
| Ethyl alcohol | 0.5 |
| Sodium alkyl sulfate* | 1.0 |
| Sodium lauroyl sarcosinate | 0.5 |
| Sucrose monolaurate | 0.1 |
| Sucrose monopalmitate | 0.1 |
| Polyoxyethylene sorbitane | 0.1 |
| monolaurate $\alpha$-Tocopherol acetate | 0.1 |
| Aluminum chlorohydroxy allantoinate | 0.01 |
| Coptis extract | 0.1 |
| Ethyl parahydroxybenzoate | 0.02 |
| Sodium benzoate | 1.0 |
| Aspartame | 0.1 |
| Flavour composition No. I | 1.0 |
| Purified water | balance |
| | 100.0% |

| Example 22: Toothpaste | |
|---|---|
| Precipitated silica | 30.0% |
| Carrageenan | 1.5 |
| Sorbitol | 15.0 |
| Glycerol | 5.0 |
| Polyethylene glycol | 5.0 |
| Sodium lauryl sulfate | 1.5 |
| Sodium lauroyl sarcosinate | 0.3 |
| Lauroyl diethanolamide | 0.7 |
| Myristoyl diethanolamide | 0.3 |
| Polyoxyethylene hardened castor oil | 0.3 |
| Tocopherol nicotinate | 0.3 |
| $\beta$-Glycyrrhetinic acid | 0.07 |
| Sodium fluoride | 0.2 |
| Phellodendron extract | 0.005 |
| Methyl parahydroxybenzoate | 0.1 |
| Sodium benzoate | 0.5 |
| Sodium saccharin | 0.1 |
| Flavour composition No. III | 1.0 |
| Purified water | balance |
| | 100.0% |

| Example 23: Toothpaste | |
|---|---|
| Aluminum hydroxide | 45.0% |
| Carrageenan | 0.3 |
| Hydroxyethyl cellulose | 1.5 |
| Sorbitol | 15.0 |
| Sodium alkyl sulfate* | 0.7 |
| Polyoxyethylene hardened castor oil | 1.0 |
| $\alpha$-Tocopherol acetate | 0.5 |
| $\beta$-Glycyrrhetinic acid | 0.04 |
| Stanous fluoride | 0.4 |
| Phellodendron extract | 1.0 |
| Butyl parahydroxybenzoate | 0.02 |
| Sodium benzoate | 0.3 |
| Sodium saccharin | 0.1 |
| Flavour composition No. II | 1.0 |
| Purified water | balance |
| | 100.0% |

| Example 24: Toothpaste | |
|---|---|
| Calcium pyrophosphate | 50.0% |
| Sodium carboxymethyl cellulose | 1.5 |
| Glycerol | 30.0 |
| Sodium lauryl sulfate | 0.5 |
| Lauroyl diethanolamide | 2.0 |
| Myristoyl diethanolamide | 0.5 |
| Tocopherol nicotinate | 0.05 |
| Sodium monofluorophosphoric acid | 0.76 |
| Phellodendron extract | 0.5 |
| Ethyl parahydroxybenzoate | 0.05 |
| Sodium benzoate | 0.5 |
| Sodium saccharin | 0.1 |
| Flavour composition No. IV | 1.5 |
| Purified water | balance |
| | 100.0% |

20

| Example 25: Toothpaste | |
|---|---|
| Insoluble sodium metaphosphate | 50.0% |
| Sodium carboxymethyl cellulose | 1.0 |
| Sorbitol | 10.0 |
| Glycerol | 20.0 |
| Sodium lauryl sulfate | 0.7 |
| Sodium lauroyl sarcosinate | 0.5 |
| Lauroyl diethanolamide | 1.5 |
| $\beta$-Glycyrrhetinic acid | 0.1 |
| Phellodendron extract | 0.05 |
| Scutellaria extract | 0.05 |
| Butyl parahydroxybenzoate | 0.1 |
| Sodium benzoate | 0.1 |
| Sodium saccharin | 0.1 |
| Flavour composition No. II | 1.0 |
| Purified water | balance |
| | 100.0% |

| Example 26: Toothpaste | |
|---|---|
| Calcium pyrophosphate | 20.0% |
| Sodium carboxymethyl cellulose | 1.0 |
| Carrageenan | 1.0 |
| Glycerol | 40.0 |
| Polyethylene glycol | 3.0 |
| Sodium lauryl sulfate | 2.0 |
| Lauroyl diethanolamide | 1.0 |
| Myristoyl diethanolamide | 0.5 |
| Coconut fatty diethanolamide | 0.5 |
| Palmitoyl diethanolamide | 0.5 |
| $\alpha$-Tocopherol acetate | 0.3 |
| $\epsilon$-Aminocaproic acid | 0.03 |
| $\beta$-Glycyrrhetinic acid | 0.02 |
| Phellodendron extract | 0.05 |
| Coptis extract | 0.05 |
| Methyl parahydroxybenzoate | 0.05 |
| Sodium benzoate | 0.5 |
| Sodium saccharin | 0.1 |
| Flavour composition No. III | 1.0 |
| Purified water | balance |
| | 100.0% |

| Example 27: Wet dentifrice | |
|---|---|
| Calcium carbonate | 70.0% |
| Sorbitol | 2.0 |
| Glycerol | 8.0 |
| Sodium lauryl sulfate | 1.5 |
| Lauroyl diethanolamide | 1.0 |
| $\alpha$-Tocopherol acetate | 0.1 |
| Phellodendron extract | 0.05 |
| Sodium saccharin | 0.1 |
| Flavour composition No. I | 2.0 |
| Purified water | balance |
| | 100.0% |

| Example 28: Liquid dentifrice | |
|---|---|
| Glycerol | 35.0% |
| Propylene glycol | 5.0 |
| Sodium polyacrylate | 2.5 |
| Sodium lauryl sulfate | 1.0 |
| Lauroyl diethanolamide | 1.5 |
| Phellodendron extract | 0.5 |
| Sodium saccharin | 0.3 |
| Flavour composition No. II | 5.0 |
| Purified water | balance |
| | 100.0% |

| Example 29: Mouthwash | |
|---|---|
| Ethyl alcohol (96%) | 20.0% |
| Polyoxyethylene hardened caster oil | 0.5 |
| Sodium lauryl sulfate | 0.5 |
| Lauroyl diethanolamide | 1.0 |
| Phellodendron extract | 0.2 |
| Sodium saccharin | 0.4 |
| Flavour composition No. IV | 3.0 |
| Purified water | balance |
| | 100.0% |

| Example 30: Pasta | |
|---|---|
| Hydroxyethyl cellulose | 4.0% |
| Sorbitol | 40.0 |
| Sodium lauryl sulfate | 0.5 |
| Lauroyl diethanolamide | 2.0 |
| Phellodendron extract | 0.4 |
| Methyl parahydroxybenzoate | 0.02 |
| Sodium saccharin | 0.02 |
| Flavour composition No. I | 1.0 |
| Purified water | balance |
| | 100.0% |

All of the oral compositions of the Examples 16 to 30 can stably retain berberine over a long term.

**Claims**

1. An oral composition comprising berberine and a flavour composition containing at least one aldehyde flavour selected from the group consisting of butanal, iso-butylaldehyde, pivalaldehyde, 2-methyl-pentanal, 2,3,5,5-tetramethylhexanal, 2-methyldecanal, tetradecanal, anisaldehyde, iso-valeric aldehyde, p-veratric aldehyde, and cinnamic aldehyde, whereas the content of straight chain aldehyde compounds having 5 to 12 carbon atoms in the flavour composition is 0.001% by weight or less, based on the total weight of the oral composition.

2. An oral composition according to claim 1, wherein the amount of the flavour composition is 0.1% to 10% by weight, based on the total weight of the oral composition.

3. An oral composition according to claim 1, wherein the berberine is derived from phellodendron extract.

4. An oral composition according to claim 3, wherein the phellodendron extract is formulated in an amount of 0.001% to 3% by weight, based on the total weight of the composition.

5. An oral composition according to claim 1, further comprising a nonionic surfactant.

6. An oral composition according to claim 5, wherein the nonionic surfactant is an alkanolamide fatty acid ester.

7. An oral composition according to claim 5, wherein the nonionic surfactant is formulated in an amount of 0.1% to 5% by weight, based on the total weight of the composition.

8. Process of preparing an oral composition comprising berberine, wherein a flavour composition containing at least one aldehyde flavour selected from the group consisting of butanal, iso-butylaldehyde, pivalaldehyde, 2-methyl-pentanal, 2,3,5,5-tetramethylhexanal, 2-methyldecanal, tetradecanal, anisaldehyde, iso-valeric aldehyde, p-veratric aldehyde and cinnamic aldehyde is added to the berberine composition, whereby the content of straight chain aldehyde compounds having 5 to 12 carbon atoms in the flavour composition is controlled to 0.001% by weight or less, based on the total weight of the oral composition.

9. Process according to claim 8, wherein the amount of the flavour composition is 0.1% to 10% by weight, based on the total weight of the oral composition.

10. Process according to claim 8, wherein the berberine is derived from phellodendron extract.

11. Process according to claim 10, wherein the phellodendron extract is formulated in an amount of 0.001% to 3% by weight, based on the total weight of the composition.

12. Process according to claim 8, wherein a nonionic surfactant is further added.

**13.** Process according to claim 12, wherein the nonionic surfactant is an alkanolamide fatty acid ester.

**14.** Process according to claim 12, wherein the nonionic surfactant is formulated in an amount of 0.1% to 5% by weight, based on the total weight of the composition.

**Patentansprüche**

**1.** Orale Zubereitung mit Berberin und einer Geschmacksmischung, enthaltend mindestens einen Aldehydgeschmacksstoff aus der Gruppe Butanal, Isobutylaldehyd, Pivalaldehyd, 2-Methylpentanal, 2,3,5,5-Tetramethylhexanal, 2-Methyldecanal, Tetradecanal, Anisaldehyd, Isovaleriansäurealdehyd, p-Veratrinsäurealdehyd und Zimtaldehyd, wobei der Gehalt der Geschmacksmischung an geradkettigen Aldehydverbindungen mit 5 - 12 Kohlenstoffatomen, bezogen auf das Gesamtgewicht der oralen Zubereitung, 0,001 Gew.-% oder weniger beträgt.

**2.** Orale Zubereitung nach Anspruch 1, dadurch gekennzeichnet, daß die Menge an der Geschmacksmischung, bezogen auf das Gesamtgewicht der oralen Zubereitung, 0,1 - 10 Gew.-% beträgt.

**3.** Orale Zubereitung nach Anspruch 1, dadurch gekennzeichnet, daß das Berberin aus Phellodendronextrakt herrührt.

**4.** Orale Zubereitung nach Anspruch 3, dadurch gekennzeichnet, daß der Phellodendronextrakt in einer Menge von 0,001 - 3 Gew.-%, bezogen auf das Gesamtgewicht der Zubereitung, formuliert ist.

**5.** Orale Zubereitung nach Anspruch 1, dadurch gekennzeichnet, daß sie zusätzlich ein nicht-ionisches Netzmittel enthält.

**6.** Orale Zubereitung nach Anspruch 5, dadurch gekennzeichnet, daß das nicht-ionische Netzmittel aus einem Alkanolamidfettsäureester besteht.

**7.** Orale Zubereitung nach Anspruch 5, dadurch gekennzeichnet, daß das nicht-ionische Netzmittel in einer Menge von 0,1 - 5 Gew.-%, bezogen auf die Gesamtmenge der Zubereitung, formuliert ist.

**8.** Verfahren zur Herstellung einer berberinhaltigen oralen Zubereitung, wobei eine Geschmacksmischung mit mindestens einem Aldehydgeschmacksstoff, ausgewählt aus Butanal, Isobutylaldehyd, Pivalaldehyd, 2-Methylpentanal, 2,3,5,5-Tetramethylhexanal, 2-Methyldecanal, Tetradecanal, Anisaldehyd, Isovaleriansäurealdehyd, p-Veratrinsäurealdehyd und Zimtaldehyd, zu der Berberinzubereitung zugegeben wird, **dadurch gekennzeichnet,** daß der Anteil an geradkettigen Aldehydverbindungen mit 5 - 12 Kohlenstoffatomen in der Geschmacksmischung, bezogen auf das Gesamtgewicht der oralen Zubereitung, auf 0,001 Gew.-% oder weniger gehalten wird.

**9.** Verfahren nach Anspruch 8, dadurch gekennzeichnet, daß die Menge an der Geschmacksmischung, bezogen auf das Gesamtgewicht der oralen Zubereitung, 0,1 - 10 Gew.-% beträgt.

**10.** Verfahren nach Anspruch 8, dadurch gekennzeichnet, daß das Berberin aus einem Phellodendronextrakt herrührt.

**11.** Verfahren nach Anspruch 10, dadurch gekennzeichnet, daß der Phellodendronextrakt in einer Menge von 0,001 - 3 Gew.-%, bezogen auf das Gesamtgewicht der Zubereitung, formuliert wird.

**12.** Verfahren nach Anspruch 8, dadurch gekennzeichnet, daß weiterhin ein nichtionisches Netzmittel zugegeben wird.

**13.** Verfahren nach Anspruch 12, dadurch gekennzeichnet, daß das nicht-ionische Netzmittel aus einem Alkanolamidfettsäureester besteht.

**14.** Verfahren nach Anspruch 12, dadurch gekennzeichnet, daß das nicht-ionische Netzmittel in einer Menge von 0,1 - 5 Gew.-%, bezogen auf das Gesamtgewicht der Zubereitung, formuliert wird.

**Revendications**

1. Composition orale comprenant de la berbérine et une composition aromatisante contenant au moins un arôme de type aldéhyde choisi parmi le butanal, l'isobutyraldéhyde, le pivalaldéhyde, le 2-méthyl-pentanal, le 2,3,5,5-tétraméthylhexanal, le 2-méthyldécanal, le tétradécanal, l'anisaldéhyde, l'aldéhyde isovalérique, l'aldéhyde p-vératrique et l'aldéhyde cinnamique, la teneur de la composition aromatisante en composés aldéhydiques à chaînes droites ayant de 5 à 12 atomes de carbone étant de 0,001 % en poids ou moins, par rapport au poids total de la composition orale.

2. Composition orale selon la revendication 1, dans laquelle la quantité de la composition aromatisante va de 0,1 à 10 % en poids, par rapport au poids total de la composition orale.

3. Composition orale selon la revendication 1, dans laquelle la berbérine provient d'extrait de philodendron.

4. Composition orale selon la revendication 3, dans laquelle l'extrait de philodendron est formulé en une quantité de 0,001 à 3 % en poids, par rapport au poids total de la composition.

5. Composition orale selon la revendication 1, comprenant en outre un tensioactif non ionique.

6. Composition orale selon la revendication 5, dans laquelle le tensioactif non ionique est un ester d'acide gras d'alcanolamide.

7. Composition orale selon la revendication 5, dans laquelle le tensioactif non ionique est formulé en une quantité de 0,1 à 5 % en poids, par rapport au poids total de la composition.

8. Procédé de préparation d'une composition orale comprenant de la berbérine, dans lequel on ajoute à la composition de berbérine une composition aromatisante contenant au moins un arôme de type aldéhyde choisi parmi le butanal, l'isobutyraldéhyde, le pivalaldéhyde, le 2-méthyl-pentanal, le 2,3,5,5-tétraméthylhexanal, le 2-méthyldécanal, le tétradécanal, l'anisaldéhyde, l'aldéhyde isovalérique, l'aldéhyde p-vératrique et l'aldéhyde cinnamique, la teneur de la composition aromatisante en composés aldéhydiques à chaînes droites ayant de 5 à 12 atomes de carbone étant ajustée à 0,001 % en poids ou moins, par rapport au poids total de la composition orale.

9. Procédé selon la revendication 8, dans lequel la quantité de la composition aromatisante va de 0,1 à 10 % en poids, par rapport au poids total de la composition orale.

10. Procédé selon la revendication 8, dans lequel la berbérine provient d'extrait de philodendron.

11. Procédé selon la revendication 10, dans lequel l'extrait de philodendron est formulé en une quantité de 0,001 à 3 % en poids, par rapport au poids total de la composition.

12. Procédé selon la revendication 8, dans lequel on ajoute encore un tensioactif non ionique.

13. Procédé selon la revendication 12, dans lequel le tensioactif non ionique est un ester d'acide gras et d'alcanolamide.

14. Procédé selon la revendication 12, dans lequel le tensioactif non ionique est formulé en une quantité de 0,1 à 5 % en poids, par rapport au poids total de la composition.